# EUROPÄISCHE PATENTSCHRIFT

(11) **EP 1 285 082 B1**
(45) Veröffentlichungstag und Bekanntmachung des Hinweises auf die Patenterteilung: **27.12.2006**
(21) Anmeldenummer: 01929648.2
(22) Anmeldetag: 16.05.2001
(51) Int. Cl.: C12P 1/00, C25B 3/04, C25B 9/00, C12M 1/00, C12P 7/22

(54) **VERFAHREN, UMFASSEND DIE INDIREKTE ELEKTROCHEMISCHE REGENERATION VON NAD(P)H**
METHOD COMPRISING THE INDIRECT ELECTROCHEMICAL REGENERATION OF NAD(P)H
PROCEDE IMPLIQUANT LA REGENERATION ELECTROCHIMIQUE INDIRECTE DE NAD(P)H

(30) Priorität: 17.05.2000 DE 10024314
(43) Veröffentlichungstag der Anmeldung: 26.02.2003
(73) Patentinhaber: BASF Aktiengesellschaft, 67056 Ludwigshafen (DE)
(72) Erfinder: STECKHAN, Eberhard, verstorben (DE); SCHMID, Andreas, CH-8049 Zürich (CH); HOLLMANN, Frank, CH-8046 Zürich (CH); HAUER, Bernhard, 67136 Fussgönheim (DE); ZELINSKI, Thomas, 67271 Neuleiningen (DE)
(74) Vertreter: Schweiger, Georg
(86) Internationale Anmeldenummer: PCT/EP2001/005601
(87) Internationale Veröffentlichungsnummer: WO 2001/088172

(56) Entgegenhaltungen:
- EP-A- 0 096 288
- EP-A- 0 099 517
- EP-A- 0 747 984
- US-A- 6 126 795
- DATABASE CHEMABS [Online] CHEMICAL ABSTRACTS SERVICE, COLUMBUS, OHIO, US; HOLLMANN, FRANK ET AL: "The first synthetic application of a monooxygenase employing indirect electrochemical NADH regeneration" retrieved from STN Database accession no. 134:262715 XP002175630 & ANGEW. CHEM., INT. ED. (2001), 40(1), 169-171 ,
- REIPA V. ET AL.: "A direct electrode-driven P450 cycle for biocatalysts" PROC. NATL. ACAD. SCI. USA, Bd. 94, 1997, Seiten 13554-13558, XP001019007 in der Anmeldung erwähnt
- HELD M. ET AL.: "An Integrated Process for the Production of Toxic Catechols from Toxic Phenols Based on a Designer Biocatalyst" BIOTECH. BIOENG., Bd. 62, Nr. 6, 1999, Seiten 641-648, XP001019008 in der Anmeldung erwähnt
- BRIELBECK B. ET AL: 'Continuous Electroenzymatic Synthesis employing the Electrochemical Enzyme Membrane Reactor' BIOCATALYSIS Bd. 10, 1994, Seiten 49 - 64, XP001017846
- RUPERT R. ET AL: 'Efficient Indirect Elektrochemical In-Situ Regeneration of NADH' TETRAHEDRON LETTERS Bd. 28, Nr. 52, 1987, Seiten 6583 - 6586, XP001017956

## Beschreibung

Die vorliegende Erfindung betrifft Verfahren, umfassend die indirekte elektrochemische Regeneration von NAD(P)H aus NAD(P)⁺, welches beispielsweise bei der enzymkatalysierten reduktiven Sauerstoffspaltung anfällt. Das erfindungsgemäße elektrochemische Regenerationsverfahren ist insbesondere anwendbar im Rahmen elektroenzymatischer, NAD(P)H verbrauchender Umsetzungen, insbesondere oxidativer enzymatischer Umsetzungen von Substraten durch Monooxygenasen. Insbesondere ist Gegenstand der Erfindung ein elektroenzymatisches Verfahren zur Monooxygenase-katalysierten Herstellung von 2,3-Dihydroxy-Phenylderivaten.

Biokatalysierte Reaktionen gewinnen sowohl in der organischen Laborsynthese als auch in zahlreichen industriellen Anwendungen immer mehr an Bedeutung. Insbesondere die gewöhnlich hohen Regio- und Stereoselektivitäten enzymatischer Umsetzungen unter gleichzeitig milden Bedingungen und hohen Ausbeuten machen sie zu attraktiven Werkzeugen in der Syntheseplanung. Im Gegensatz zu den hydrolytischen Enzymen, die bereits vielfältige Verwendung finden, ist der Einsatz von Redoxenzymen für enantioselektive Reduktionen und chemo-, regio- und enantioselektive Oxidationen trotz ihres großen synthetischen Potenzials noch nicht sehr weit verbreitet. Grund dafür ist vor allem das bisher nicht zufriedenstellend gelöste Problem einer effektiven Cofaktorregeneration. Neben den etablierten Verfahren der enzymgekoppelten Cofaktorregenerierung [1a,b,c,d] wurden inzwischen auch elektrochemische Methoden entwickelt und auf NAD(P)⁺- und NAD(P)H-abhängige Enzyme angewandt [2a,b,c]. Der Vorteil der indirekten elektrochemischen Cofaktorregenerierung besteht darin, daß lediglich das Produktionsenzym benötigt wird und sich somit die häufig schwierige Optimierung eines Doppel-Enzym-Systems erübrigt. Darüber hinaus kann auf ein Cosubstrat verzichtet werden.

Monooxygenasen besitzen eine hohe synthetische Bedeutung, da sie in der Lage sind, Sauerstofffunktionen regio- und stereoselektiv in ihre Substrate einzubauen. Dafür benötigen sie molekularen Sauerstoff, dessen O-O-Bindung reduktiv unter Bildung von Wasser gespalten wird [3a,b]. Die nativen Cofaktoren der Monooxygenasen NADH oder NADPH liefern dazu die notwendigen Reduktionsäquivalente. Die bisherigen in vitro-Verfahren mit Monooxygenasen als Produktionsenzyme beruhen auf einer enzymgekoppelten Cofaktorregenerierung unter Verwendung der Formiat-Dehydrogenase [4a,b] (für NADH bzw. NADPH) sowie der Glucose-6-phosphat-Dehydrogenase [5] (für NADPH).

Reipa et al. beschreiben in [10] ein Verfahren zur elektrochemischen Regeneration von Putidaredoxin, dem natürlichen Redoxpartner der Cytochrom CYP 101-Monooxygenase (E.C.1.14.15.1). Hierzu wird die Verwendung einer speziellen Antimon-dotierten Zinnoxidelektrode vorgeschlagen, welche zur Putidaredoxin-Reduktion geeignet ist.

Held et al. beschreiben in [8] die biokatalytische Produktion von 3-Phenylkatechol unter Verwendung ganzer Zellen von Escherichia coli JM 101 (pHDT 461). Dieser rekombinante Mikroorganismus ist zur Produktion des Enzyms 2-Hydroxybiphenyl-3-monooxygenase ausgelegt. Eine elektrochemische Regeneration von verbrauchtem NADH in in diesem Zusammenhang nicht erforderlich, da der Cofaktor durch das enzymatische System der intakten E. coli-Zellen wieder regeneriert wird.

EP-A-99517, EP-A-96288 und EP-A-747984 beschreiben die elektrochemische Regeneration von NAD⁺ zu NADH, jedoch wird das NAD⁺ im Rahmen eines mikrobiellen/enzymatischen Reduktionsreaktion hergestellt.

Aufgabe der vorliegenden Erfindung war es, ein enzymfreies, selektives und effektives Verfahren zur indirekten elektrochemischen NAD(P)H-Regenerierung zu entwickeln, welches z.B. gekoppelt mit Monooxygenasen zur oxidativen Umsetzung von Substraten unter reduktiver Sauerstoffspaltung geeignet ist. Eine weitere Aufgabe bestand in der Bereitstellung eins Verfahrens, das die enzymatische, Monooxygenase-katalysierte Synthese von 2,3-Dihydroxyphenyl-Verbindungen, wie 2,3,-Dihydroxybiphenyl, unter indirekter elektrochemischer NAD(P)H-Regenerierung ermöglicht.

### Kurze Beschreibung der Erfindung

Diese Aufgaben wurden gelöst durch Bereitstellung eines elektroenzymatischen Verfahrens zur Herstellung von 2,3-Dihydroxyphenylderivaten der allgemeinen Formel I 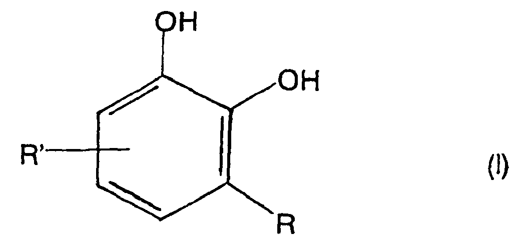 worin R für gegebenenfalls ein- oder mehrfach substituiertes Phenyl, C₁-C₆-Alkyl, Halogen oder CN steht; und
R' für H oder OH steht;
wobei man bei diesem Verfahren
a) eine Monohydroxyphenyl-Verbindung der allgemeinen Formel II 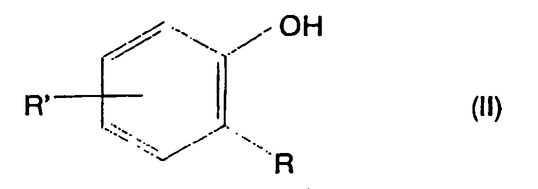 worin R und R' die oben angegebenen Bedeutungen besitzen, mit 2-Hydroxybiphenyl-3-monooxygenase (HbpA) (E.C. 1.14.13.44) in Gegenwart von NADH und Sauerstoff umsetzt; und
b) das gebildete NAD⁺ elektrochemisch zu NADH reduziert.

Die Aufgaben werden außerdem gelöst durch Bereitstellung eines Verfahrens zur NAD(P)H-Regeneration bei einer NAD(P)H-verbrauchenden oxidativen enzymatischen Umsetzung eines Substrates, bei dem man eine NAD(P)H-verbrauchende oxidative enzymatischen Umsetzung eines oxidierbaren Substrates in Gegenwart von NAD(P)H und vorzugsweise unter Sauerstoffverbrauch durchführt, und das bei der Oxidation des Substrats gebildete NAD(P)⁺ elektrochemisch zu NAD(P)H reduziert. Beispielsweise kann man eine NAD(P)H-abhängige Monooxygenase (aus der Klasse E.C. 1.14.-.-) mit dem oxidierbaren Substrat in Gegenwart von NAD(P)H und in Anwesenheit von Sauerstoff inkubieren und das bei reduktiver Sauerstoffspaltung und oxidation des Substrats gebildete NAD(P)⁺ elektrochemisch zu NAD(P)H reduzierten.

Die vorliegende Erfindung erlaubt erstmalig die enzymungebundene, indirekte elektrochemische Regeneration von NADH bzw. NADPH im Rahmen von Sauerstoff verbrauchenden, z.B. Monooxygenase-katalysierter Reaktionen. Aufgrund der indirekten elektrochemischen Cofaktorregeneration ist eine kontinuierliche Substratumsetzung möglich.

Eine "indirekte" elektrochemische Regeneration von NAD(P)H im Sinne der vorliegenden Erfindung ist gegeben, wenn der Cofaktor über einen geeigneten Redoxkatalysator regeneriert wird, welcher die zur Reduktion erforderlichen Elektronen von der Kathode auf den oxidierten Cofaktor überträgt.

### Detaillierte Beschreibung der Erfindung

Ein erster Gegenstand der Erfindung betrifft ein elektroenzymatisches Verfahren zur Herstellung von 2,3-Dihydroxyphenylderivaten der allgemeinen Formel I 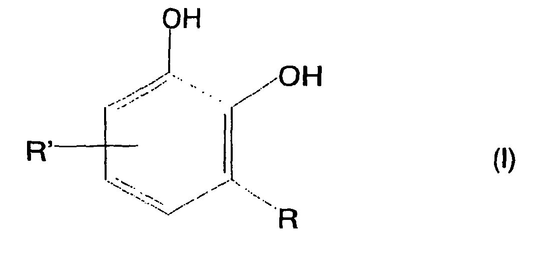 worin R für gegebenenfalls ein oder mehrfach, z.B. durch Halogen, wie z.B. F, Cl, Br oder J, CN oder OH, insbesondere OH, substituiertes Phenyl; für C₁-C₆-Alkyl, wie z.B. Methyl, Ethyl, n- oder i- Propyl, n-, i- oder t-Butyl, sowie n-Pentyl oder n-Hexyl und jeweils die verzweigten Analoga davon; für Halogen, wie z.B. F, Cl, Br oder J; oder für CN steht; und R' für H oder OH steht,
wobei R' in m- oder p-Position, vorzugsweise p-Position zur 2-Hydroxygruppe des Phenylrings steht,
und wobei man
a) eine Monohydroxyphenyl-Verbindung der allgemeinen Formel II 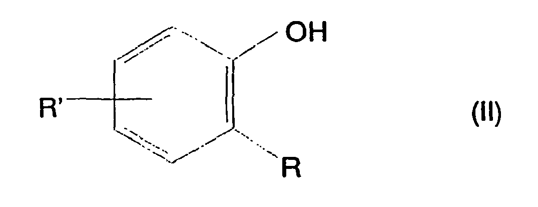 worin R und R' die oben angegebenen Bedeutungen besitzen,
   mit 2-Hydroxybiphenyl-3-monooxygenase (HbpA) (E.C. 1.14.13.44) in Gegenwart von NADH und Sauerstoff umsetzt; und
b) das gebildete NAD⁺ elektrochemisch zu NADH reduziert.

Bevorzugt führt man die elektrochemische NAD⁺-Reduktion in Gegenwart eines Hydridorhodium-Redoxkatalysators durch, der kathodisch herstellbar und regenerierbar ist. Der Redoxkatalysator ist dabei ein vorzugsweise löslicher Rhodiumkomplex, der bei einem Kathodenpotenzial im Bereich von -650 bis -800 mV, gemessen gegen Ag/AgCl(gesättigt) (pH=6-9; T=20-60°C, insbesondere etwa 20 bis 35°C, wie z.B. etwa 30°C) elektrochemisch in den Hydridorhodium-Komplex überführbar ist.

Besonders bevorzugt verwendet man zur Durchführung der HbpA-katalysierten Reaktion einen Rhodium-Komplex der allgemeinen Formel III

[Cp Rh (III)(bpy)Cl]Cl (III)

worin
- Cp: für Cyclopentadienyl oder Pentamethylcyclopentadienyl steht und
- bpy: für 2,2'-Bipyridyl steht, wobei jeder der Pyridylringe gegebenenfalls ein- oder mehrfach, insbesondere einfach, durch eine Donorgruppe substituiert ist, wobei die Donorgruppe ausgewählt ist unter Methyl, Methoxy und Acetamido. Bevorzugt kann jeder Pyridylring einen dieser Substituenten in 4- oder 5-Position tragen. Insbesondere sind die Pyridylringe mit gleichen Donorgruppen substituiert.

Im Rahmen des erfindungsgemäßen Verfahrens unter Beteiligung der HbpA-Katalyse wird der Rhodium-Komplex der Formel III kathodisch zu einem Hydridorhodium-Komplex der Formel IIIa

[Cp Rh (I)(bpy)H]Cl

mit H als Proton
oder

[Cp Rh (III)(bpy)H]Cl (IIIa)

mit H als Hydridion
worin Cp und bpy die oben für Formel III angegebenen Bedeutungen besitzen,
reduziert, welcher dann zur NAD⁺-Reduktion befähigt ist.

Die erfindungsgemäße Herstellung der Dihydroxyverbindungen der allgemeinen Formel I wird bevorzugt unter folgenden Bedingungen durchgeführt:
a) Substratkonzentration (Verbindung der Formel II): 0,01 bis 50 mM, insbesondere 0,1 bis 4 mM;
b) NAD⁺ Konzentration: 0,01 bis 5 mM, insbesondere 0,01 bis 0,5 mM;
c) Rhodium-Komplex-Konzentration: 1 µM bis 5 mM, insbesondere 5 µM bis 0,5 mM;
d) HbpA-Konzentration: 1 bis 5000 U/l, insbesondere 10 bis 1000 U/l;
e) FAD-Konzentration: 0 bis 200 µM, insbesondere 0 bis 20 oder 1 bis 20 µM;
f) Catalase-Konzentration: 0 bis 1 x 10⁷ U/l;
g) pH: 4 bis 9, insbesondere 6 bis 7,5
h) Temperatur:10 bis 40°C, insbesondere 20 bis 35°C oder etwa 30°C
i) Kathodenpotenzial: -600 bis -900mV, insbesondere -650 bis -800 mV
j) Sauerstoffeintrag: 20 bis 120 cm³/(min·l), durch Einblasen oder insbesondere blasenfrei über Sauerstoff-permeable Membranen oder Schläuche, wie z.B. beschrieben in [11].

Erfindungsgemäß brauchbare Elektrodensysteme sind z.B. beschrieben in [12] und [13]. Repräsentative, nichtlimitierende Beispiele für geeignete Kathoden-/Anodenpaare sind: Kohlekathode/Platinanode, wie insbesondere zylinderförmige Kohlekathode (Kohlefilz, Sigraflex^{®})/Platindrahtanode.

Die erfindungsgemäß umgesetzten Substrate der Formel II sind allgemein zugängliche Verbindungen und entweder käuflich erhältlich oder mit üblichen Methoden der organischen Chemie herstellbar. Als nicht limitierende Beispiele können genannt werden:

2-Hydroxy-C₁-C₆-alkylbenzole, 2-Hydroxy-halogenbenzole, 2-Hydroxybenzonitril, und die 2,5-Dihydroxyanaloga dieser Benzolderivate; 2-Hydroxybiphenyl und mehrfach hydroxylierte Biphenyle, wie z.B. 2,4-, 2,5- oder 2,6-Dihydroxybiphenyl; 2,n'-Dihydroxybiphenyle (mit n' = 2, 3 oder 4); oder 2,n',m'-Trihydroxybiphenyle (wobei n' und m' voneinander verschieden sind und jeweils für 2, 3 oder 4 stehen).

Als Redoxkatalysatoren werden erfindungsgemäß bevorzugt [CpRh(bpy)Cl]Cl-Komplexe verwendet. Die Herstellung dieser Komplexe ist allgemein bekannt und erfolgt wie beschrieben in [14] oder [15]. Die nach elektrochemischer Reduktion bei -700 mV (vs. Ag/AgCl_{ges.}) oder aber auch chemisch mit Formiat daraus gebildeten Hydridorhodiumkomplexe überführen NAD(P)⁺ schnell und quantitativ in die enzymaktive 1,4-NAD(P)H-Form-[2,6]

Als repräsentatives Beispiel für brauchbare Enzyme kann, stellvertretend für die Klasse flavinabhängiger Monooxygenasen, die 2-Hydroxybiphenyl-3-monooxygenase (HbpA, E.C. 1.14.13.44) aus P. azelaica, die NADH als Cofaktor benötigt, genannt werden [7]. Das Enzym liegt als Homotetramer mit einer Gesamtmasse von 256 kDa vor und katalysiert entsprechend Schema 1 die selektive ortho-Hydroxylierung einer Reihe α-substituierter Phenolderivate. Auf chemischem Weg ist diese Reaktion mit vergleichbarer Selektivität nicht durchführbar.

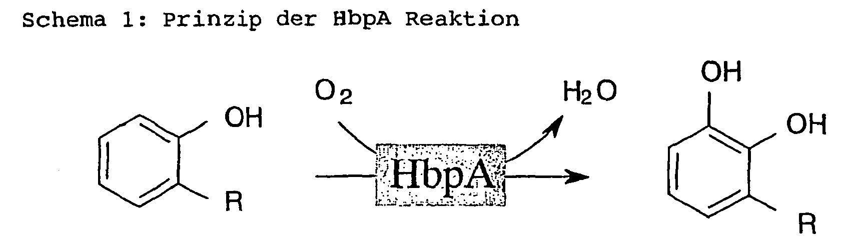

Die HbpA-katalysierte Reaktion erfolgt vorzugsweise in einem wässrigen Reaktionsmedium, dessen pH-Wert mit üblichen, die Umsetzung und den elektrochemischen Prozess nicht negativ beeinflussenden Puffersubstanzen, wie z.B. HEPES, PIPES und insbesondere Kaliumphosphat-Puffer und Tris/HCl-Puffer, auf einen geeigneten Wert eingestellt wurde. Die Pufferkonzentration liegt dabei im Bereich von etwa 20 mM bis 0,2 M, insbesondere etwa 20 bis 50 mM. Der pH-Wert wurde bevorzugt auf etwa 6 bis 8, insbesondere etwa 7,5 eingestellt.

Das Reaktionsmedium kann weitere übliche Zusätze, wie z.B. Lösungsvermittler für das Substrat, Cofaktoren, wie z.B. FAD oder FMN, für das eingesetzte Enzym, und dergleichen enthalten.

Bei oxidationsempfindlichen Enzymsystemen ist gegebenenfalls die Verwendung von Antioxidantien sinnvoll. Kommt es z.B. verfahrensbedingt zur Bildung von Wasserstoffperoxid, welches die Enzymaktivität negativ beeinträchtigen kann, so kann man die Reaktion in Gegenwart von Catalase, z.B. zugesetzt in einer Konzentration von 1·10⁵ U/l, durchführen.

Ein weiterer Gegenstand der Erfindung betrifft ein Verfahren zur elektrochemischen NAD(P)H-Regeneration, anwendbar bei einer NAD(P)H-verbrauchenden oxidativen enzymatischen Umsetzung eines Substrates, wobei man eine NAD(P)H-verbrauchende oxidative enzymatischen Umsetzung eines oxidierbaren Substrates in Gegenwart von NAD(P)H und vorzugsweise unter Sauerstoff-Verbrauch durchführt, und das bei der Oxidation des Substrats gebildete NAD(P)⁺ elektrochemisch zu NAD(P)H reduziert. Dieses Verfahren eignet sich bevorzugt zur Durchführung im Rahmen Monooxygenase-katalysierter Reaktionen. Eine NAD(P)H-abhängige Monooxygenase (aus der Klasse E.C. 1.14.-.-) wird dabei mit dem oxidierbaren Substrat in Gegenwart von NAD(P)H und in Anwesenheit von Sauerstoff inkubiert und das bei reduktiver Sauerstoffspaltung und oxidation des Substrats gebildete NAD(P)⁺ elektrochemisch zu NAD(P)H reduziert.

Gemäß einer bevorzugten Verfahrensvariante führt man die elektrochemische NAD(P)⁺-Reduktion in Gegenwart eines vorzugsweise löslichen Hydridorhodium-Redoxkatalysators durch, der kathodisch herstellbar und regenerierbar ist.

Das erfindungsgemäße Verfahren zur NAD(P)H-Regeneration verwendet als Redoxkatalysator vorzugsweise einen Rhodiumkatalysator, der bei einem Kathodenpotenzial im Bereich von -650 bis -800 mV, gemessen gegen Ag/AgCl(gesättigt) (pH=6-9; T=20-60°C, insbesondere etwa 20 bis 35°C, wie z.B. etwa 30°C) elektrochemisch in den Hydridorhodium-Komplex überführbar ist.

Bevorzugt sind beim erfindungsgemäßen Verfahren zur NAD(P)H-Regeneration Rhodium-Komplexe der allgemeinen Formel III'

[Cp Rh (III)(bpy)Cl]Cl (III')

einsetzbar, worin
- Cp: für Cyclopentadienyl oder Pentamethylcyclopentadienyl steht und
- bpy: für 2,2'-Bipyridyl steht, wobei jeder der Pyridylringe gegebenenfalls ein- oder mehrfach, insbesondere einfach, durch eine Donorgruppe substituiert ist, wobei die Donorgruppe ausgewählt ist unter Methyl, Methoxy und Acetamido. Weiterhin kann als Donorgruppe ein von Polyethylenglycol, wie z.B. von PEG 2000 bis 20000, abgeleiteter Rest enthalten sein. Bevorzugt kann jeder Pyridylring einen dieser Substituenten in 4- oder 5-Position tragen. Insbesondere sind die Pyridylringe mit gleichen Donorgruppen substituiert.

Der Rhodium-Komplex der Formel III' wird kathodisch zu einem Hydridorhodium-Komplex der Formel IIIa'

[Cp Rh (I)(bpy)H]Cl

mit H als Proton
oder

[Cp Rh (III)(bpy)H]Cl (IIIa')

mit H als Hydridion
worin Cp und bpy die für Formel III' angegebenen Bedeutungen besitzen, reduziert, welcher zur NAD⁺-Reduktion befähigt ist.

Das erfindungsgemäße Verfahren zur NAD(P)H-Regeneration wird bevorzugt unter folgenden Verfahrensbedingungen durchgeführt:
a) NAD(P)+ Konzentration: 0,01 bis 5 mM, insbesondere 0,01 bis 0,5 mM;
b) Rhodium-Komplex-Konzentration: 1 µM bis 5 mM, insbesondere 5 µM bis 0,5 mM;
c) Monooxygenase-Konzentration: 1 bis 5000 U/l, insbesondere 10 bis 1000 U/l;
d) Cofaktor-Konzentration (wie z.B. FAD): 0 bis 200 µM;, insbesondere 0 bis 20 oder 1 bis 20 µM;
e) Catalase-Konzentration: 0 bis 1·10⁷ U/l;
f) pH: 4 bis 9, insbesondere 6 bis 7,5
g) Temperatur: 10 bis 40°C, insbesondere 20 bis 35°C oder etwa 30°C;
h) Kathodenpotenzial: -600 bis -900mV, insbesondere -650 bis -800 mV
i) Sauerstoffeintrag: 20 bis 120 cm³/(min·l) durch Einblasen oder insbesondere blasenfrei über Sauerstoff-permeable Membranen oder Schläuche, wie z.B. beschrieben in [11].

Die erfindungsgemäß umgesetzten Substrate sind allgemein zugängliche Verbindungen und entweder käuflich erhältlich oder mit üblichen Methoden der organischen Chemie herstellbar.

Als Redoxkatalysatoren werden erfindungsgemäß bevorzugt [CpRh(bpy)Cl]Cl-Komplexe verwendet. Die Herstellung dieser Komplexe ist allgemein bekannt und erfolgt wie beschrieben in [14] oder [15]. Die nach elektrochemischer Reduktion bei -700 mV (vs. Ag/AgCl_{ges}.) oder aber auch chemisch mit Formiat daraus gebildeten Hydridorhodiumkomplexe überführen NAD(P)⁺ schnell und quantitativ in die enzymaktive 1,4-NAD(P)H-Form [2,6].

Die Reaktion erfolgt vorzugsweise in einem wässrigen Reaktionsmedium, dessen pH-Wert mit üblichen, die Umsetzung und den elektrochemischen Prozess nicht negativ beeinflussenden Puffersubstanzen, wie z.B. HEPES, PIPES und insbesondere Kaliumphosphat-Puffer und Tris/HCl-Puffer, auf einen geeigneten Wert eingestellt wurde. Die Pufferkonzentration liegt dabei im Bereich von etwa 20 mM bis 0,2 M, insbesondere etwa 20 bis 50 mM. Der pH-Wert wurde bevorzugt auf etwa 6 bis 8, insbesondere etwa 7,5 eingestellt.

Das Reaktionsmedium kann weitere übliche Zusätze, wie z.B. Lösungsvermittler für das Substrat, Cofaktoren, wie z.B. FAD oder FMN, für das eingsetzte Enzym, und dergleichen enthalten.

Bei oxidationsempfindlichen Enzymsystemen ist die Verwendung von Antioxidantien sinnvoll. Kommt es z.B. verfahrensbedingt zur Bildung von Wasserstoffperoxid, welches die Enzymaktivität negativ beeinträchtigen kann, so kann man die Reaktion in Gegenwart von Catalase, z.B. zugesetzt in einer Konzentration von 1·10⁵ U/l, durchführen.

Das erfindungsgemäße Verfahren zur NAD(P)B-Regeneration ist bevorzugt einsetzbar für die folgenden, eine oxidative enzymatische Umsetzung umfassenden Reaktionstypen:
a) Oxidation an gesättigten oder ungesättigten, aliphatischen oder aromatischen Kohlenstoffatomen, insbesondere durch Hydroxylierung, Epoxidierung und Baeyer-Villiger-Oxidatioa;
b) Schwefel- oder Selen-Oxidation;
c) Stickstoff- oder Phosphor-Oxidation;
d) Oxidation von Halogeniden.

**Nichtlimitierende Beispiele für Reaktionstyp a):**
(1) Hydroxylierung am aliphatischen Kohlenstoff: 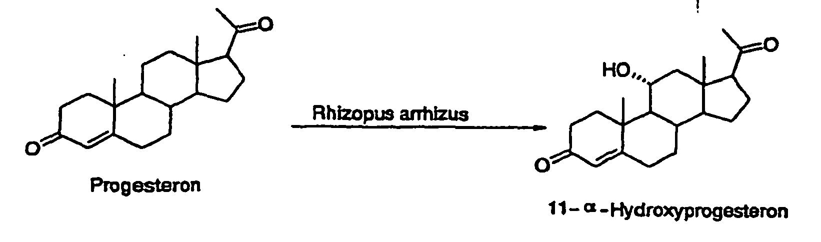 z.B. beschrieben in JP 75/54957 (Takeda)
(2) ω-Hydroxylierung langkettiger Fettsäuren, katalysiert durch Cytochrom P450-Monooxygenase; z.B. beschrieben in der DE-A-199 35 115.5 (BASF AG)
(3) Hydroxylierung allylischer oder benzylischer Kohlenstoffatome 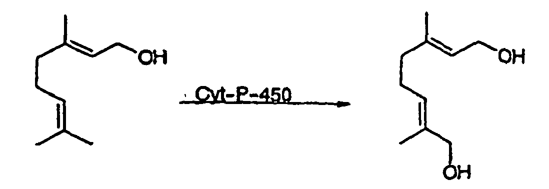 H. Fretz. W. D. Woggon, R. Voges *Hel, Chim. Acta,* **1989**., *72,* 391-400 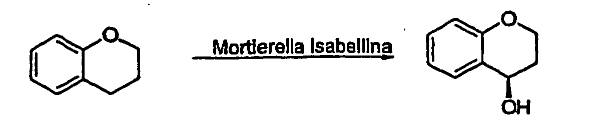 H. L Holland, T. S. Manoharan, F. Schweizer *Tetrahedron: Asymmetry,* **1991**, *2,* 335-338
(4) Epoxidierung: 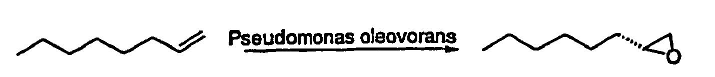 S. W. May, B. J. Abbot *J. Biol. Chem,* **1973** *,248 ,* 1725-1730
(5) Baeyer-Villiger-Oxidation: 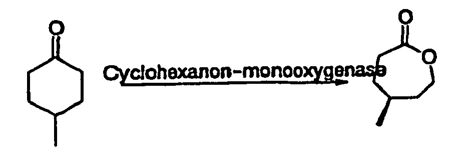 Roberts et al. *J. Mol. Cat. B Enzymatic,* **1998**, *4,* 111 ff
(6) Beteroaromatenoxidation: 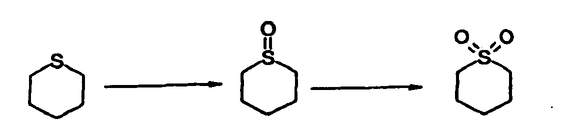 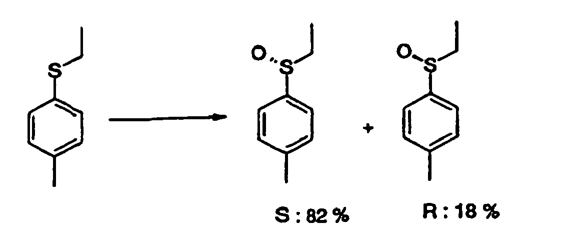 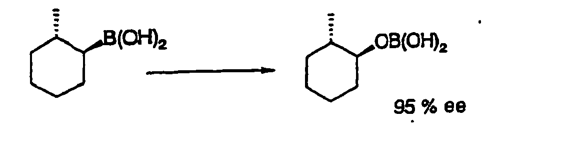
jeweils katalysiert durch Cyclohexanon-Monooxygenase; beschrieben von Walsh, C.T., et al Angew. Chem., 1988, 100, 242; und Roberts, et al., J. Mol. Cat. B Enzymatic, 1998, 4, 111.

Gegenstand der Erfindung ist außerdem die Verwendung eines Redoxkatalysators gemäß obiger Definition zur kontinuierlichen oder diskontinuierlichen elektrochemischen Regenerierung von NAD(P)H, bevorzugte bei Sauerstoff verbrauchenden, insbesondere bei Monooxygenase-katalysierten Oxidationsreaktionen, insbesondere Oxidationsreaktionen des oben bezeichneten Typs.

Schließlich betrifft die vorliegende Erfindung Bioreaktoren zur kontinuierlichen oder diskontinuierlichen Durchführung Sauerstoff verbrauchender, insbesondere Monooxygenase-katalysierter elektroenzymatischer Reaktionen, umfassend in einem Reaktionsraum ein Elektrodenpaar sowie ein flüssiges, ein- oder zweiphasiges Reaktionsmedium, welches Enzym, insbesondere Monooxygenase, Substrat, NAD(P)H-Kofaktor und eine Redoxkatalysator jeweils gemäß obiger Definition enthält, wobei an der Kathode ein Elektrodenpotenzial anliegt, welches zur Übertragung von Redoxäquivalenten (Elektronen) auf den Redoxkatalysator geeignet ist.

Bioreaktoren geeigneten Typs sind z.B. beschrieben in [16] und [17], worauf hiermit Bezug genommen wird.

Der Betrieb des Reaktors und die Verfahrensführung können vom Fachmann den jeweiligen Erfordernissen der gewünschten Redoxreaktion angepaßt werden. Ein- oder zweiphasige Reaktionsmedien sind ebenfalls anwendbar wie eine Kompartimentierung des Reaktionsraums. zweiphasige Reaktionssysteme sind z.B. vorteilhaft bei Umsetzung von Substraten und/oder Bildung von Produkten, welche im wässrigen Reaktionsmedium nicht oder nur schlecht löslich sind. Dabei kann man z.B. Substrat in der organischen Phase vorlegen. Dieses wird kontinuierlich in die wassrige Phase abgegeben, dort umgesetzt und das gebildete Produkt gegebenenfalls wieder an die organische Phase abgegeben. Eine Kompartimentierung erlaubt beispielsweise eine räumliche und zeitliche Trennung von Enzymreaktion und Elektrodenreaktion. Bevorzugt ist außerdem ein sauerstoffeintrag durch Begasung, insbesondere blasenfreie Begasung, wie z.B. beschrieben von Rissom in [4b], worauf hiermit Bezug genommen wird.

Die Erfindung wird nun unter Bezugnahme auf die beiliegenden Figuren näher erläutert. Dabei zeigt:

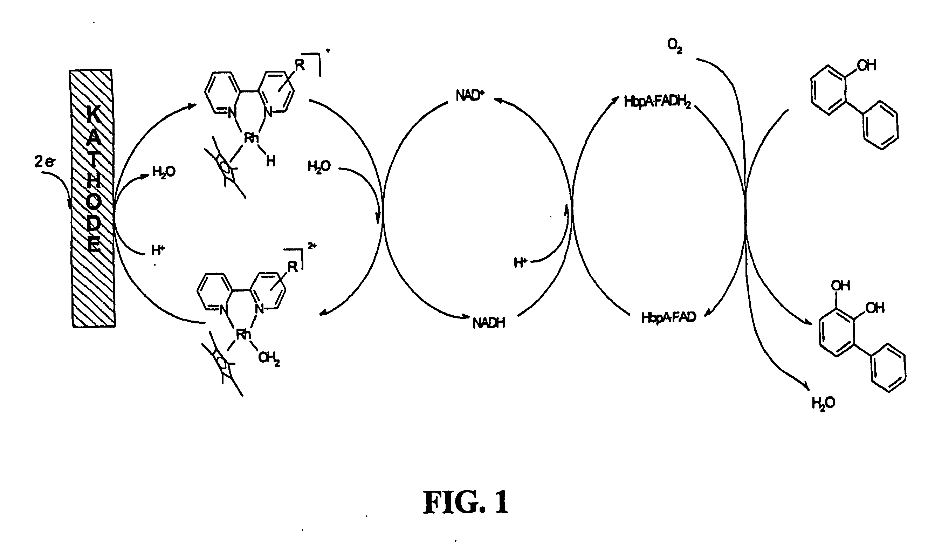

- Figur 1: eine schematische Darstellung des erfindungsgemäßen elektroenzymatischen Prozesses der Bildung von 2,3-Dihydroxybiphenyl unter gleichzeitiger elektrochemischer NADH-Regeneration; dabei wird kathodisch der Hydridorhodium(III)-Redoxkatalysator gebildet und regeneriert. Nach Übertragung des Hydridions auf NAD⁺ und Bildung von NADH reduziert dieses die Monooxygenase, wie z.B. die prosthetische FAD-Gruppe der 2-Hydroxybiphenyl-3-monooxygenase, zur aktiven FADH₂-Funktion. Diese reduzierte Form des Enzyms katalysiert dann in Gegenwart von Sauerstoff die Oxygenierung des Substrates, wie z.B. von 2-Hydroxybiphenyl zu 2,3-Dihydroxybiphenyl.
- Figur 2: den Einfluß von O₂ auf die NADH-Bildung unter indirekt elektrochemischer Regeneration (■ ohne Drucklufteintrag; ○ mit Drucklufteintrag von 10 cm³/min)
- Figur 3: den postulierten Mechanismus der Hydridoxidation
- Figur 4: den Einfluß des Sauerstoffgehaltes auf die Bildung von 2,3-Dihydroxybiphenyl unter indirekter elektrochemischer Cofaktorregenerierung (○: kein Sauerstoffeintrag in die Lösung; ■: nach 1h einsetzender Sauerstoffeintrag)
- Figur 5: einen Vergleich der Umsatzgeschwindigkeit unter chemischer und indirekt-elektrochemischer Hydridorhodiumbildung (○: redoxkatalytische, chemische Regeneration mit Formiat als Reduktionsmittel; ■: indirekte elektrochemische Regeneration)
- Figur 6: in schematischer Darstellung einen geeigneten Batchraktor, umfassend ein Reaktionsgefäß mit Rührer, Ringkathode, mittig angeordneter Anode, Referenzelektrode, Druckluftzufuhr in das Reaktionsmedium.

### Beispiel 1: Elektroenzymatische Oxidation von 2-Hydroxybiphenyl zu 2,3-Dihydroxybiphenyl

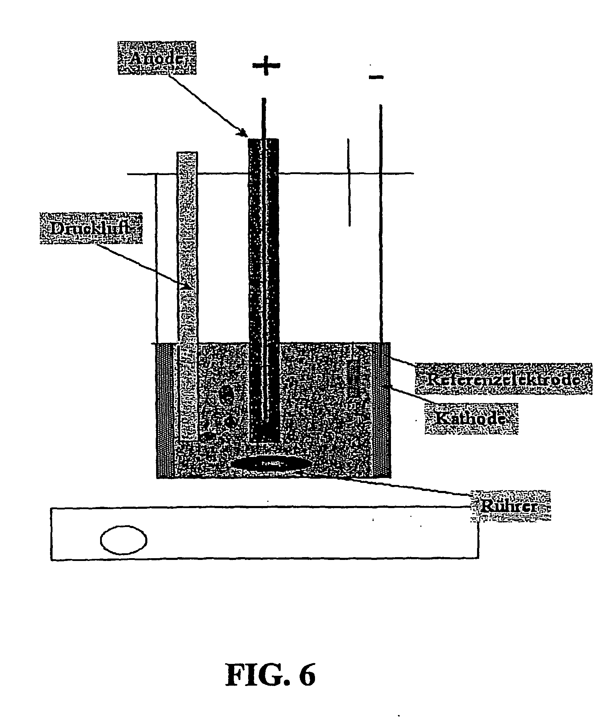

Entsprechend Figur 1 wird die elektroenzymatischen Umsetzung in einer Batch-Elektrolysezelle, schematisch dargestellt in Figur 6, durchgeführt. Als Kathode dient hierbei eine zylindrische Kohlefilzelektrode (Volumen ca. 27 cm³). Durch Ummantelung der Platin-Gegenelektrode mit einem Dialyseschlauch (Ausschlußgewicht 10 kDa) werden die Bedingungen einer geteilten Zelle erreicht.

Das Kathodenpotenzial von -750 mV wird gegen eine Ag/AgCl_{ges.}-Schlauchelektrode eingestellt. In 100 ml KPi-Puffer (50 mM, pH 7.5) sind der oxidierte Cofaktor NAD⁺ (0.2 mM), [Cp*Rh(bpy)Cl]²⁺ (0.1 mM), FAD (20 µM), Catalase (250000 U), HbpA (19 U) sowie das Substrat (2 mM) gelöst. Die Umsetzung erfolgt bei T=30°C und über einen Zeitraum von 5 Stunden.

Der Reaktionsfortschritt wird mittels HPLC-Chromatographie an einer RP-18-Säule mit Methanol/Wasser (0.1% H₃PO₄) 60:40 als Eluens verfolgt.

### Beispiel 2: Einfluß von gelöstem Sauerstoff auf die NADH-Regeneration

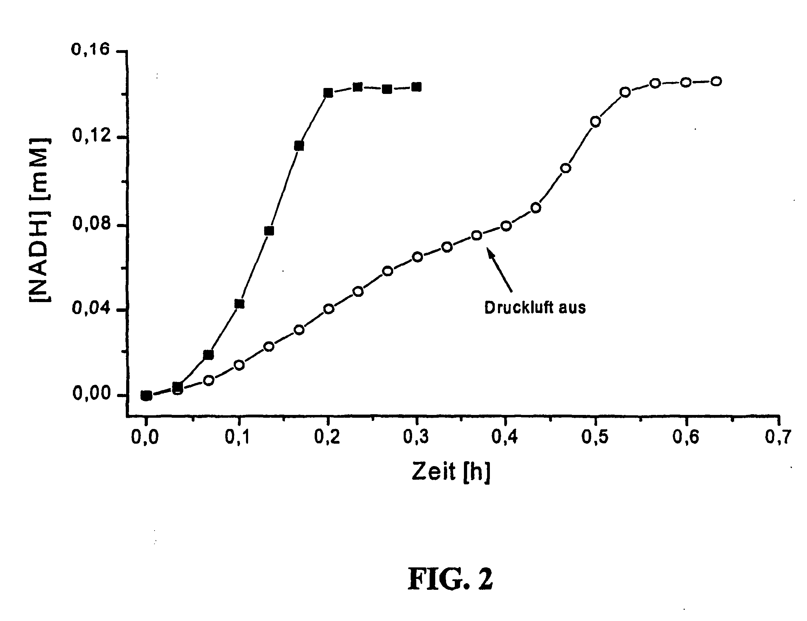

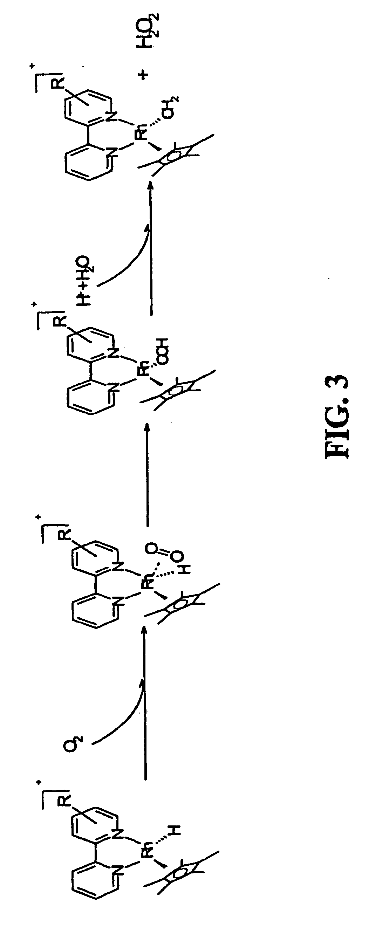

Da Sauerstoff ein Bestandteil der Reaktionssequenz ist, muß der Einfluß des gelösten Sauerstoffs auf die Komponenten des Systems untersucht werden. Dabei zeigt sich, daß Sauerstoff die NADH-Erzeugung sowohl durch den chemisch mit Formiat als auch durch den erfindungsgemäß auf elektrochemischem Weg gebildeten Hydridorhodiumkomplex inhibiert (Figur 2). Wie aus Figur 2 deutlich wird, geht bei einem Sauerstoffeintrag von beispielsweise 10 cm³/min die NADH-Bildungsgeschwindigkeit von 1.1 ^{mmol}/_{1.h} auf 0.27 ^{mmol}/_{1.h} zurück. Bei einem Eintrag von 15 cm³/min ist keine NADH-Bildung mehr nachweisbar. Die Inhibierung ist jedoch reversibel, da nach Unterbrechung des Sauerstoffstromes die NADH-Bildungsgeschwindigkeit fast wieder ihren optimalen Wert erreicht. Ebenso erreicht die NADH-Konzentration ihren Maximalwert. Als Produkt der Reaktion des Hydridorhodiumkomplexes mit molekularem Sauerstoff konnte Wasserstoffperoxid nachgeweisen werden, dessen Bildung wie in Figur 3 angegeben denkbar ist. Darüber hinaus wird Wasserstoffperoxid bei dem anliegenden Potential auch durch direkte Reduktion des Sauerstoffs an der Kathode gebildet. Daher ist es zweckmäßig Catalase zuzusetzen, da diese während der enzymatischen Umsetzung Wasserstoffperoxid zerstört.

### Beispiel 3: Einfluß von Sauerstoffzufuhr auf den Umsatz

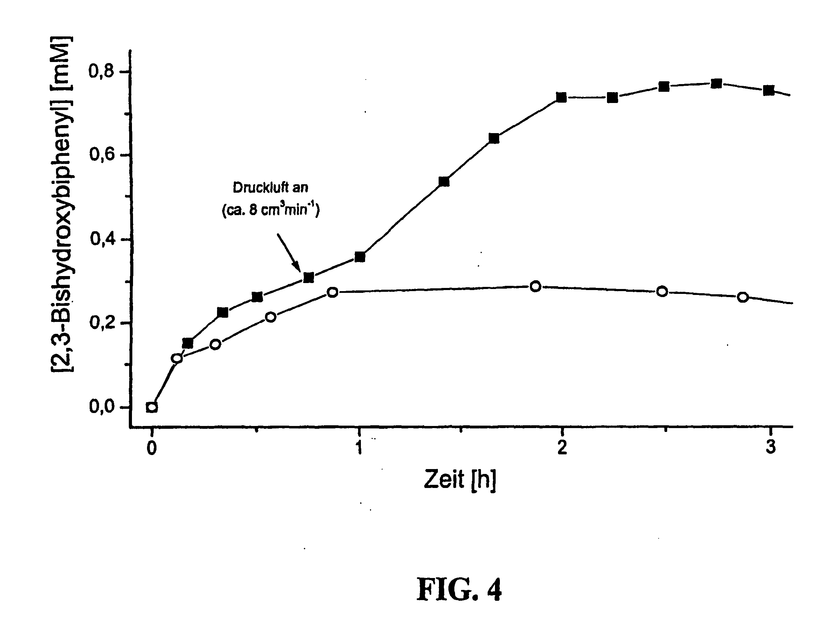

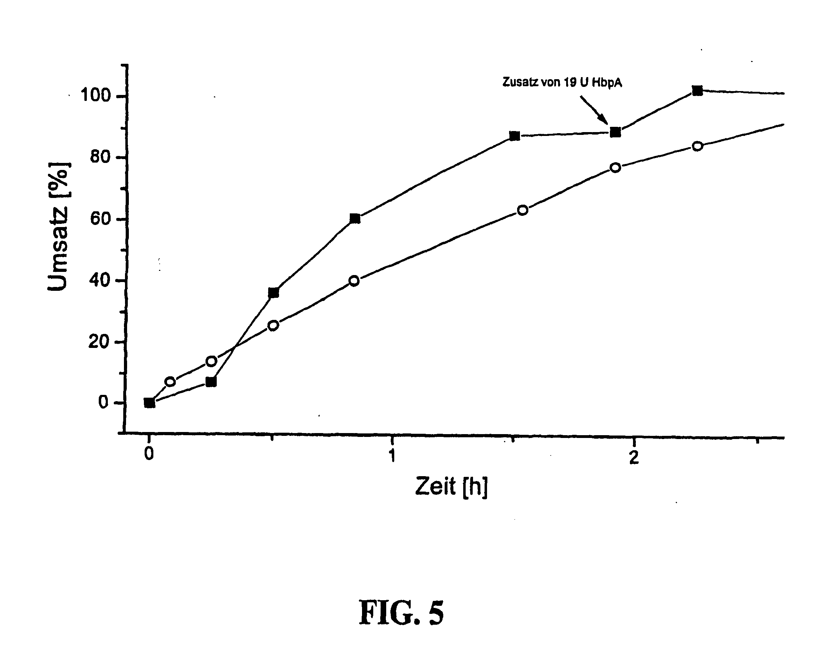

Die Ergebnisse in Figur 4 zeigen, daß der Umsatz bei Reaktionen ohne externe Sauerstoffzuführung nach kurzer Zeit bei ca. 20% stagniert (offene Kreise bzw. gefüllte Quadrate bis ca. lh). Mit nach 1h einsetzender Sauerstoffzufuhr (8 cm³/min) steigt die ' Umsatzgeschwindigkeit und damit die Produktbildung bis zu einem Wert von 1.1 ^{mmol}/_{1.h} (202 ^{mg}/_{1.h}) an. Die vom Mediator erbrachten Wechselzahlen liegen hierbei bei 11 h⁻¹. Ähnliche Werte werden erreicht, wenn der Hydridorhodiumkomplex chemisch mit Natriumformiat erzeugt wird. In Figur 5 sind die Umsatzgeschwindigkeiten für die indirekte elektrochemische Cofaktorregenerierung unter den genannten Bedingungen, jedoch unter einem ständigen Sauerstoffeintrag von 10 cm³/min und für den durch Formiat (c(NaHCO₂) = 160 mM; ansonsten gleiche Bedingungen) getriebenen redoxkatalytischen Prozess dargestellt. Die Produktivität liegt bei ca. 50% des bereits optimierten fermentativen bzw. des in vitro-Verfahrens unter enzymgekoppelter NADH-Regeneration (390 ^{mg}/_{1.h}) [8].

Die Reaktionsgeschwindigkeit ist nicht durch die Bildung des Hydridorhodiumkomplexes an der Kathode limitiert, sondern durch die kompetitive Hemmung des Redoxkatalysators durch die Konkurrenzreaktion mit molekularem Sauerstoff (Figur 4).

In dem erfindungsgemäßen kontinuierlichen elektroenzymatischen Verfahren sollte sich der negative Einfluß des gelösten Sauerstoffs auf die indirekt elektrochemische NADH-Regeneration leicht durch eine Kompartimentierung des Gesamtsystems in einzelne Module ausschalten lassen. Durch die räumlich und zeitlich auf den elektrochemischen Schritt folgende Zudosierung des für die Enzymreaktion unabdingbaren Sauerstoffs wird dessen inhibitorischer Effekt auf die NADH-Regenerierung minimiert.

Die Langzeitstabilität der durchgeführten Batchelektrolysen kann durch Verringerung der denaturierenden Wirkung der nicht-blasenfreien Begasung verbessert werden. An der Phasengrenze flüssig/gasförmig treten nämlich starke Scherkräfte auf, die mittelfristig zur Enzymdenaturierung führen. In Vergleichsexperimenten konnte gezeigt werden, daß HbpA bei Thermostatisierung bei 30°C und Rühren (u = 250 min⁻¹) auch nach 12 h noch mehr als 85% ihrer Initialaktivität besitzt. Mit einsetzender Druckluftzufuhr nimmt diese in Abhängigkeit von der zufuhrgeschwindigkeit um bis zu 70% innerhalb einer Stunde ab. Durch Verwendung eines Enzym-Membran-Reaktors [9] können das Produktionsenzym vor dem negativen Einfluß des heterogenen Sauerstoffeintrages geschützt und folglich hohe Prozeßlaufzeiten erreicht werden. Insbesondere durch Anwendung einer zweiphasigen Reaktionsführung und der resultierenden in situ Zudosierung des Substrates und Extraktion des Catecholproduktes sollte ein effektiver kontinuierlicher Prozeß verwirklichbar sein.

Erfindungsgemäß konnte erstmalig eine durch eine flavinabhängige Monooxygenase katalysierte Umsetzung unter indirekt elektrochemischer NADH-Regeneration erfolgreich durchführen.

Die vorliegende Erfindung schafft die Grundlage für die Einbindung von Monooxygenasen in organische Synthesen sowohl im Labormaßstab, als auch in der industriellen Anwendung. Diese Klasse oxidierender Enzyme ist synthetisch hoch interessant, da sie beispielsweise Hydroxyfunktionen in aromatische Systeme und auch in nicht aktivierte reine Kohlenwasserstoffe einführen können. Sie können außerdem Sauerstoff auf Heteroatome oder auf Doppelbindungen unter Bildung von Epoxiden übertragen. Zusätzlich katalysieren sie Baeyer-Villiger-Oxidationen. In allen Fällen sind enantiomerenreine Produkte zugänglich.

Das zugrundeliegende elektrochemische NAD(P)H-Regenerationskonzept stellt eine effektive und einfach anwendbare Alternative zu den bisherigen in vivo-Verfahren bzw. solchen Verfahren, die sich eines enzymatischen Regenerationsystems bedienen, dar. Die allgemeine Anwendbarkeit, auch unter den Umsatzbedingungen sauerstoffabhängiger Monooxygenasen, konnte belegt werden.

### Literaturverzeichnis

[1] a) Hummel,W., et al., Eur. J. Biochem. 1989, 184, 1-13; b) Shaked, Z., et al., J. Am. Chem. Soc. 1980, 102, 7104-7108; c) Abril, O., et al., Bioorg. Chem. 1989, 17, 41-52; d) Seelbach, K., et al., Tetrahedron Lett. 1996, 37, 1377-1380
[2] a) Hilt, G., et al., Liebigs Ann./Recueil 1997, 2289-2296; b) Westerhausen, D., et al., Angew. Chem. 1992, 104, 1496-1498; Angew. Chem. Int. Ed. Engl. 1992, 31, 1529 - 1531 c) Ruppert, R., et al Tetrahedron Lett. 1987, 52(28), 6583-6586
[3] a) Walsh, C.T. Acc. Chem. Res. 1980, 13, 148-155; b) Walsh, C.T. et al., Angew. Chem. 1988, 100, 342-352; Angew. Chem. Int. Ed. Engl. 1988
[4] a) Hummel, W., et al.; Appl. Microbiol Biotechnol. 1986, 25, 175-185; b) Rissom, R., et al., Tetrahedron Asymmetry 1997, 15(8), 2523-2526
[5] Wong, C.-H., et al., J. Am. Chem. Soc. 1981, 103, 4890-4899
[6] a) Steckhan, E., et al., Organometallics, 1991, 10, 1568-1577; b) Ruppert, R., et al., J. Chem. Soc., Chem. Commun., 1988, 1150-1151
[7] Suske,W. A., et al., J. Biol. Chem., 1997, 272 (39), 24257-242565
[8] Held, M., et al., Biotechnol. Bioeng., 1999, 62 (6), 641-648
[9] Wandrey, C., Chem. Ing. Tech. 1976, 48, 537
[10]Reipa, V., et al., Proc.Natl. Acad. Sci. USA, 1997, 94, 13554-13558
[11]Schneider et al., Enzyme Microbiol. Technol., 1995, 17, 839
[12]Sawyer, D.T., Electrochemistry for Chemists, 2. Aufl, Wiley-Interscience; New York
[13]Kissinger, P. T., Laboratory Techniques in Electroanalytical Chemistry, Marcel Dekker, Inc.; New York/ Basel
[14]Kölle, U., et al., Angew. Chem. 1987, 99, 572
[15]Kölle, U., et al., Chem.Ber. 1989, 122, 1869
[16]Kragl, U., et al.,, Chem. Ing. Tech., 1992, 499
[17]Brielbeck, B., et al., Biocatalysis, 1994, 10, 49

## Patentansprüche

1. Elektroenzymatisches Verfahren zur Herstellung von 2,3-Dihydroxyphenylderivaten der allgemeinen Formel I 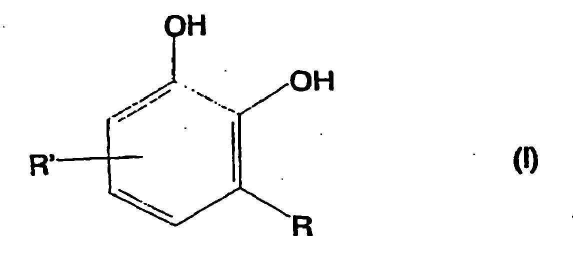 worin
R für gegebenenfalls ein- oder mehrfach substituiertes Phenyl, C₁-C₆-Alkyl, Halogen oder CN steht; und
R' für H oder OH steht;
**dadurch gekennzeichnet, dass** man
a) eine Monohydroxyphenyl-Verbindung der allgemeinen Formel II 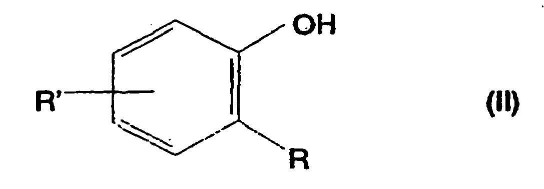 worin R und R' die oben angegebenen Bedeutungen besitzt, mit 2-Hydroxybiphenyl-3-monooxygenase (HbpA) (E.C.1.14.13.44) in Gegenwart von NADH und Sauerstoff umsetzt; und
b) das gebildete NAD⁺ elektrochemisch zu NADH reduziert.

2. Verfahren nach Anspruch 1, **dadurch gekennzeichnet, dass** man die elektrochemische NAD⁺-Reduktion in Gegenwart eines Hydridorhodium-Redoxkatalysators durchführt, der kathodisch herstellbar und regenerierbar ist.

3. Verfahren nach Anspruch 2, **dadurch gekennzeichnet, dass** man als Redoxkatalysator einen Rhodiumkomplex verwendet, der bei einem Kathodenpotenzial im Bereich von -650 bis -800 mV, gemessen gegen Ag/AgCl(gesättigt) (pH=6-9; T=20-30°C) elektrochemisch in den Bydridorhodium-Komplex überführbar ist.

4. Verfahren nach Anspruch 3, **dadurch gekennzeichnet, dass** man einen Rhodium-Komplex der allgemeinen Formel III
[Cp Rh (III)(bpy)Cl]Cl (III)
einsetzt, worin
Cp für Cyclopentadienyl oder Pentamethylcyclopentadienyl steht und
bpy für 2,2'-Bipyridyl steht, wobei jeder der Pyridylringe gegebenenfalls ein- oder mehrfach durch eine Donorgruppe substituiert ist.

5. Verfahren nach Anspruch 4, **dadurch gekennzeichnet, dass** der Rhodium-Komplex der Formel III kathodisch zu einem Hydridorhodium-Komplex der Formel IIIa
[Cp Rh (I)(bpy)H]Cl (IIIa)
reduziert wird, welcher zur NAD⁺-Reduktion befähigt ist.

6. Verfahren nach einem der Ansprüche 1 bis 5, **dadurch gekennzeichnet, dass** es unter folgenden Verfahrensbedingungen durchgeführt wird:
a) Substratkonzentration: 0,1 bis 4 mM;
b) NAD⁺ Konzentration: 0,01 bis 0,5 mM;
c) Rhodium-Komplex-Konzentration: 5 µM bis 0,5 mM;
d) BbpA-Konzentration: 10 bis 1000 U/l;
e) FAD-Konzentration: 0 bis 200 µM;
f) Catalase-Konzentration: 0 bis 1-10⁷ U/l;
g) pH: 6 bis 7,5
h) Temperatur: 20 bis 30°C
i) Kathodenpotenzial: -650 bis -800 mV
j) Sauerstoffeintrag: 20 bis 120 cm³/(min·l)

7. Verfahren zur elektrochemischen NAD(P)H-Regeneration aus enzymatisch gebildetem NAD(P)⁺
**dadurch gekennzeichnet, dass** man
eine NAD(P)B-verbrauchende oxidative enzymatische Umsetzung eines oxidierbaren Substrates in Gegenwart von NAD(P)N durchführt, und das bei der Oxidation des Substrats gebildete NAD(P)⁺ elektrochemisch zu NAD(P)H reduziert, wobei man für die enzymatische Umsetzung eine NAD(P)H-abhängige Monooxygenase (aus der Klasse E.C. 1.14.-.-) mit dem oxidierbaren Substrat in Gegenwart von NAD(P)H und in Anwesenheit von Sauerstoff inkubiert und das bei reduktiver Sauerstoffspaltung und Oxidation des Substrats gebildete NAD(P)⁺ elektrochemisch zu NAD(P)H reduziert.

8. Verfahren nach Anspruch 7, **dadurch gekennzeichnet, dass** man die elektrochemische NAD(P)⁺-Reduktion in Gegenwart eines Hydridorhodium-Redoxkatalysators durchführt, der kathodisch herstellbar und regenerierbar ist.

9. Verfahren nach Anspruch 8, **dadurch gekennzeichnet, dass** man als Redoxkatalysator einen Rhodiumkatalysator verwendet, der bei einem Kathodenpotenzial im Bereich von -650 bis -800 mV, gemessen gegen Ag/AgCl(gesättigt) (pH = 6-9; T = 20-35°C) elektrochemisch in den Hydridorhodium-Komplex überführbar ist.

10. Verfahren nach Anspruch 9, **dadurch gekennzeichnet, dass** man einen Rhodium-Komplex der allgemeinen Formel III
[Cp Rh (III)(bpy)Cl]Cl (III)
einsetzt, worin
Cp für Cyclopentadienyl oder Pentamethylcyclopentadienyl steht und
bpy für 2,2'-Bipyridyl steht, wobei jeder der Pyridylringe gegebenenfalls ein- oder mehrfach durch eine Donorgruppe substituiert ist.

11. Verfahren nach Anspruch 10, **dadurch gekennzeichnet, dass** der Rhodium-Komplex der Formel III kathodisch zu einem Hydridorhodium-Komplex der Formel IIIa
[Cp Rh (I)(bpy)H]Cl (IIIa)
reduziert wird, welcher zur NAD⁺-Reduktion befähigt ist.

12. Verfahren nach einem der Ansprüche 7 bis 11, **dadurch gekennzeichnet, dass** es unter folgenden Verfahrensbedingungen durchgeführt wird:
a) NAD(P)⁺ Konzentration: 10 µM bis 0,5 mM;
b) Rhodium-Komplex-Konzentration: 5 µM bis 0,5 mM;
c) Monooxygenase-Konzentration: 10 bis 1000 U/l;
d) FAD-Konzentration: 0 bis 200 µM;
e) Catalase-Konzentration: 0 bis 1·10⁷ U/l;
f) pH: 5 bis 9
g) Temperatur: 20 bis 35°C
h) Kathodenpotenzial: -650 bis -800 mV
i) Sauerstoffeintrag: Sauerstoffeintrag: 20 bis 120 cm³/min·l

13. Verfahren nach einem der Ansprüche 7 bis 12, **dadurch gekennzeichnet, dass** die oxidative enzymatische Umsetzung einen der folgenden Reaktionstypen umfasst:
a) Oxidation an gesättigten oder ungesättigten aliphatischen oder aromatischen Kohlenstoffatomen, insbesondere durch Hydroxylierung, Epoxidierung und Baeyer-Villiger-Oxidation;
b) Schwefel- oder Selen-Oxidation;
c) Stickstoff- oder Phosphor-Oxidation;
d) Oxidation von Halogeniden.

14. Verwendung eines Redoxkatalysators gemäß der Definition in einem der Ansprüche 8 bis 11 zur kontinuierlichen oder diskontinuierlichen elektrochemischen Regenerierung von NAD(P)H bei Monooxygenase-katalysierten Oxidationsreaktionen.

15. Verwendung nach Anspruch 14, wobei die Oxidationsreaktion ausgewählt ist unter:
a) Oxidation an gesättigten oder ungesättigten aliphatischen oder aromatischen Kohlenstoffatomen, insbesondere durch Hydroxylierung, Epoxidierung und Baeyer-Villiger-Oxidation;
b) Schwefel- oder Selen-Oxidation;
c) Stickstoff- oder Phosphor-Oxidation;
d) Oxidation von Halogeniden.

16. Bioreaktor, zur kontinuierlichen oder diskontinuierlichen Durchführung Monoxygenase-katalysierter elektroenzymatischer Reaktionen, umfassend in einem Reaktionsraum ein Elektrodenpaar sowie ein flüssiges Reaktionsmedium, welches Monooxygenase, Substrat, NAD(P)H-Kofaktor und eine Redoxkatalysator gemäß der Definition in einem der Ansprüche 2 bis 5 enthält, wobei an der Kathode ein Elektrodenpotential anliegt, welches zur Übertragung von Redoxäquivalenten (Elektronen) auf den Redoxkatalysator geeignet ist.

17. Bioreaktor nach Anspruch 16, **dadurch gekennzeichnet, dass** das flüssige Reaktionsmedium ein- oder zweiphasig ist.

## Claims

1. An electroenzymatic method for preparing 2,3-dihydroxyphenyl derivatives of the formula I 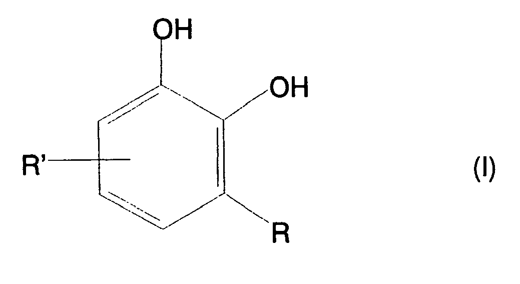 where
R is unsubstituted, monosubstituted or polysubstituted phenyl, C₁-C₆ alkyl, halogen or CN; and
R' is H or OH;
which comprises
a) converting a monohydroxyphenyl compound of the formula II 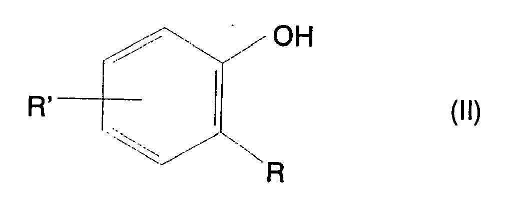 where R and R' have the abovementioned meanings, using 2-hydroxybiphenyl 3-monooxygenase (HbpA) (E.C.1.14.13.44) in the presence of NADH and oxygen; and
b) reducing the NAD⁺ formed to NADH electrochemically.

2. A method as claimed in claim 1, wherein the electrochemical NAD⁺ reduction is carried out in the presence of a hydridorhodium redox catalyst which can be prepared cathodically and can be regenerated.

3. A method as claimed in claim 2, wherein the redox catalyst used is a rhodium complex which can be converted electrochemically into the hydridorhodium complex at a cathode potential in the range from -650 to -800 mV, measured against Ag/AgCl(saturated) (pH=6-9; T=20-30°C).

4. A method as claimed in claim 3, wherein a rhodium complex of the formula III
[Cp Rh (III)(bpy)Cl]Cl (III)
is employed, where
Cp is cyclopentadienyl or pentamethylcyclopentadienyl and
bpy is 2,2'-bipyridyl, each of the pyridyl rings being unsubstituted or mono- or polysubstituted by a donor group.

5. A method as claimed in claim 4, wherein the rhodium complex of the formula III is cathodically reduced to give a hydridorhodium complex of the formula IIIa
[Cp Rh (I)(bpy)H]Cl (IIIa),
which is capable of reducing NAD⁺.

6. A method as claimed in any of claims 1 to 5, which is carried out under the following conditions:
a) substrate concentration: 0.1 to 4 mM;
b) NAD⁺ concentration: 0.01 to 0.5 mM;
c) rhodium complex concentration: 5 µM to 0.5 mM;
d) HbpA concentration: 10 to 1 000 U/l;
e) FAD concentration: 0 to 200 µM;
f) catalase concentration: 0 to 1·10⁷ U/l;
g) pH: 6 to 7.5
h) temperature: 20 to 30°C
i) cathode potential: -650 to -800 mV
j) oxygen input: 20 to 120 cm³/(min·l)

7. A method for electrochemical NAD(P)H regeneration from enzymatically formed NAD(P)⁺,
wherein
an NAD(P)H-consuming oxidative enzymatic conversion of an oxidizable substrate is carried out in the presence of NAD(P)H, and the NAD(P)⁺ formed in the course of oxidation of the substrate is electrochemically reduced to NAD(P)H, and wherein for performing the enzymatic reaction an NAD(P)H-dependent monooxygenase (of class E.C. 1.14.-.-) is incubated with the oxidizable substrate in the presence of NAD(P)H and in the presence of oxygen, and the NAD(P)⁺ formed in the course of the reductive cleavage of oxygen and oxidation of the substrate is electrochemically reduced to NAD(P)H.

8. A method as claimed in claim 7, wherein the electrochemical NAD(P)⁺ reduction is carried out in the presence of a hydridorhodium redox catalyst which can be prepared cathodically and can be regenerated.

9. A method as claimed in claim 8, wherein the redox catalyst used is a rhodium catalyst which can be converted electrochemically into the hydridorhodium complex at a cathode potential in the range from -650 to -800 mV, measured against Ag/AgCl(saturated) (pH=6-9; T=20-35°C).

10. A method as claimed in claim 9, wherein a rhodium complex of the formula III
[Cp Rh (III)(bpy)Cl]Cl (III)
is employed, where
Cp is cyclopentadienyl or pentamethylcyclopentadienyl and
bpy is 2,2'-bipyridyl, each of the pyridyl rings being unsubstituted or mono- or polysubstituted by a donor group.

11. A method as claimed in claim 10, wherein the rhodium complex of the formula III is cathodically reduced to give a hydridorhodium complex of the formula IIIa
[Cp Rh (I)(bpy)H]Cl (IIIa)
which is capable of reducing NAD⁺.

12. A method as claimed in any of claims 7 to 11, which is carried out under the following conditions:
a) NAD(P)⁺ concentration: 10 µM to 0.5 mM;
b) rhodium complex concentration: 5 µM to 0.5 mM;
c) monooxygenase concentration: 10 to 1 000 U/l;
d) FAD concentration: 0 to 200 µM;
e) catalase concentration: 0 to 1·10⁷ U/l;
f) pH: 5 to 9
g) temperature: 20 to 35°C
h) cathode potential: -650 to -800 mV
i) oxygen input: 20 to 120 cm³/(min·l)

13. A method as claimed in any of claims 7 to 12, wherein the oxidative enzymatic conversion comprises one of the following reaction types:
a) oxidation of saturated or unsaturated aliphatic or aromatic carbon atoms, in particular by hydroxylation, epoxidation and Baeyer-Villiger oxidation;
b) sulfur or selenium oxidation;
c) nitrogen oxidation or phosphorus oxidation;
d) oxidation of halides.

14. The use of a redox catalyst as defined in any of claims 8 to 11 for the continuous or discontinuous electrochemical regeneration of NAD(P)H in monooxygenase-catalyzed oxidation reactions.

15. The use as claimed in claim 14, wherein the oxidation reaction is selected from:
a) oxidation of saturated or unsaturated aliphatic or aromatic carbon atoms, in particular by hydroxylation, epoxidation and Baeyer-Villiger oxidation;
b) sulfur or selenium oxidation;
c) nitrogen oxidation or phosphorus oxidation;
d) oxidation of halides.

16. A bioreactor for carrying out in a continuous or discontinuous manner monooxygenase-catalyzed electroenzymatic reactions, which comprises in a reaction chamber a pair of electrodes and a liquid reaction medium comprising monooxygenase, substrate, NAD(P)H cofactor and a redox catalyst as defined in any of claims 2 to 5, with an electrode potential, which is suitable for transferring redox equivalents (electrons) to the redox catalyst, being present at the cathode.

17. A bioreactor as claimed in claim 16, wherein the liquid reaction medium is mono- or biphasic.

## Revendications

1. Procédé électroenzymatique de préparation de dérivés 2,3-dihydroxyphényliques de la formule générale I : 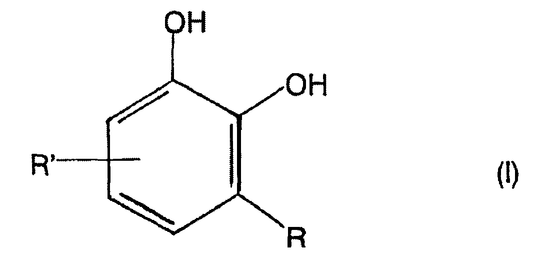 dans laquelle
R représente un groupe phényle éventuellement substitué à une ou à plusieurs reprises ou alkyle en C₁-C₆, de l'halogène ou CN, et
R' représente H ou OH,
**caractérisé en ce que**
a) on fait réagir, en présence de NADH et d'oxygène, un composé monohydroxyphénylique de la formule générale II : 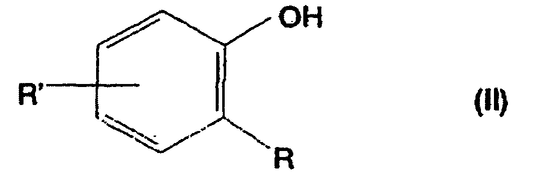 dans laquelle R et R' ont les significations indiquées ci-dessus, avec de la 2-hydroxybiphényl-3-monooxygénase (HbpA) (E.C.1.14.13.44), et
b) on réduit le NAD⁺ formé en NADH par voie électrochimique.

2. Procédé suivant la revendication 1, **caractérisé en ce qu'**on effectue la réduction électrochimique de NAD⁺ en présence d'un catalyseur d'oxydoréduction à base d'hydridorhodium qui, par voie cathodique, est préparable et régénérable.

3. Procédé suivant la revendication 2, **caractérisé en ce que**, comme catalyseur d'oxydoréduction, on utilise un complexe de rhodium qui est convertissable par voie électrochimique en le complexe d'hydridorhodium à un potentiel de cathode de l'ordre de -650 à -800 mV, mesuré contre Ag/AgCl (saturé) (pH=6-9; T=20-30°C).

4. Procédé suivant la revendication 3, **caractérisé en ce qu'**on met en oeuvre un complexe de rhodium de la formule générale III :
[Cp Rh (III)(bpy)Cl]Cl (III)
dans laquelle
Cp représente un groupe cyclopentadiényle ou pentaméthylcyclopentadiényle, et
bpy représente un groupe 2,2'-bipyridyle, chacun des noyaux pyridyle étant éventuellement substitué à une ou à plusieurs reprises par un groupe donneur.

5. Procédé suivant la revendication 4, **caractérisé en ce que** le complexe de rhodium de la formule III est réduit par voie cathodique en un complexe d'hydridorhodium de la formule IIIa :
[Cp Rh (I)(bpy)H]Cl (IIIa)
qui est approprié pour la réduction de NAD⁺.

6. Procédé suivant l'une des revendications 1 à 5, **caractérisé en ce qu'**il est effectué dans les conditions opératoires suivantes :
a) concentration du substrat : 0,1 à 4 mM,
b) concentration en NAD⁺ : 0,01 à 0,5 mM,
c) concentration en complexe de rhodium : 5 µM à 0,5 mM,
d) concentration en HbpA : 10 à 1000 U/l,
e) concentration en FAD : 0 à 200 µM,
f) concentration en catalase : 0 à 1·10⁷ U/l,
g) pH : 6 à 7,5,
h) température : 20 à 30°C,
i) potentiel de cathode : -650 à -800 mV,
j) introduction d'oxygène : 20 à 120 cm³/(min·l).

7. Procédé de régénération électrochimique de NAD(P)H à partir de NAD(P)⁺ formé par voie enzymatique,
**caractérisé en ce qu'**on effectue une réaction enzymatique par oxydation, consommant de NAD(P)H, d'un substrat oxydable en présence de NAD(P)H et on réduit par voie électrochimique le NAD(P)⁺ formé au cours de l'oxydation du substrat en NAD(P)H, une monooxygénase dépendant de NAD(P)H (de la classe E.C.1.14.-.-) étant, pour la réaction enzymatique, mise à incuber avec le substrat oxydable en présence de NAD(P)H et d'oxygène et le NAD(P)⁺ formé au cours de la séparation d'oxygène par réduction et de l'oxydation du substrat étant réduit en NAD(P)H par voie électrochimique.

8. Procédé suivant la revendication 7, **caractérisé en ce qu'**on effectue la réduction électrochimique de NAD(P)⁺ en présence d'un catalyseur d'oxydoréduction à base d'hydridorhodium, qui, par voie cathodique, peut être préparé et régénéré.

9. Procédé suivant la revendication 8, **caractérisé en ce que**, comme catalyseur d'oxydoréduction, on utilise un catalyseur à base de rhodium qui, à un potentiel de cathode de l'ordre de -650 à -800 mV, mesuré contre Ag/AgCl (saturé) (pH=6-9; T=20-35°C), peut être converti par voie électrochimique en le complexe d'hydridorhodium.

10. Procédé suivant la revendication 9, **caractérisé en ce qu'**on met en oeuvre un complexe de rhodium de la formule générale III :
[Cp Rh (III) (bpy)Cl]Cl (III)
dans laquelle
Cp représente un groupe cyclopentadiényle ou pentaméthylcyclopentadiényle, et
bpy représente un groupe 2,2'-bipyridyle, chacun des noyaux pyridyle étant éventuellement substitué à une ou à plusieurs reprises par un groupe donneur.

11. Procédé suivant la revendication 10, **caractérisé en ce que** le complexe de rhodium de la formule III est réduit par voie cathodique en un complexe d'hydridorhodium de la formule IIIa :
[Cp Rh (I) (bpy)H]Cl (IIIa)
qui est approprié pour la réduction de NAD⁺.

12. Procédé suivant l'une des revendications 7 à 11, **caractérisé en ce qu'**il est effectué dans les conditions opératoires suivantes :
a) concentration en NAD(P)⁺ : 10 µM à 0,5 mM,
b) concentration en complexe de rhodium : 5 µM à 0,5 mM,
c) concentration en monooxygénase : 10 à 1000 U/l,
d) concentration en FAD : 0 à 200 µM,
e) concentration en catalase : 0 à 1·10⁷ U/l,
f) pH : 5 à 9,
g) température: 20 à 35°C,
h) potentiel de cathode : -650 à -800 mV,
i) introduction d'oxygène : 20 à 120 cm³/(mim·l).

13. Procédé suivant l'une des revendications 7 à 12, **caractérisé en ce que** la réaction enzymatique par oxydation comprend un des types de réaction suivants :
a) oxydation sur des atomes de carbone aromatiques ou aliphatiques saturés ou insaturés, en particulier par hydroxylation, époxydation et oxydation de Baeyer-Villiger,
b) oxydation de soufre ou de sélénium,
c) oxydation d'azote ou de phosphore,
d) oxydation d'halogénures.

14. Utilisation d'un catalyseur d'oxydoréduction suivant la définition d'une des revendications 8 à 11, pour la régénération électrochimique continue ou discontinue de NAD(P)H dans le cas de réactions d'oxydation catalysées par de la monooxygénase.

15. Utilisation suivant la revendication 14, dans laquelle la réaction d'oxydation est choisie parmi:
a) une oxydation sur des atomes de carbone aromatiques ou aliphatiques saturés ou insaturés, en particulier par hydroxylation, époxydation et oxydation de Baeyer-Villiger,
b) une oxydation de soufre ou de sélénium,
c) une oxydation d'azote ou de phosphore,
d) une oxydation d'halogénures.

16. Bioréacteur pour la mise en oeuvre continue ou discontinue de réactions électroenzymatiques catalysées par de la monooxygénase, comprenant, dans une chambre réactionnelle, une paire d'électrodes ainsi qu'un milieu réactionnel liquide qui contient de la monooxygénase, un substrat, un cofacteur NAD(P)H et un catalyseur d'oxydoréduction suivant la définition de l'une des revendications 2 à 5, un potentiel d'électrode, qui est approprié pour le transfert d'équivalents d'oxydoréduction (électrons) sur le catalyseur d'oxydoréduction, étant appliqué à la cathode.

17. Bioréacteur suivant la revendication 16, **caractérisé en ce que** le milieu réactionnel liquide est à une ou deux phases.
